# EUROPEAN PATENT SPECIFICATION

(11) **EP 3 150 229 B1**
(45) Date of publication and mention of the grant of the patent: **07.03.2018**
(21) Application number: 15729209.5
(22) Date of filing: 15.05.2015
(51) Int. Cl.: A61K 47/10, A61K 47/34

(54) **SYNERGISTIC SURFACTANT COMPOSITION**
SYNERGISTISCHE SURFACTANT ZUSAMMENSETZUNG
COMPOSITION SYNERGIQUE DE SURFACTANTS

(30) Priority: 26.05.2014 ES 201430782
(43) Date of publication of application: 05.04.2017
(73) Proprietor: Sipcam Inagra S.A., 46009 Valencia (ES)
(72) Inventor: VALIERI, Gianluca, E-46009 Valencia (ES)
(74) Representative: Rizzo, Sergio
(86) International application number: PCT/ES2015/070388
(87) International publication number: WO 2015/181414

(56) References cited:
- WO-A2-2011/093656
- GB-A- 2 406 859
- US-A1- 2004 221 765
- DATABASE WPI Week 199707 Thomson Scientific, London, GB; AN 1997-073055 XP002745046, & JP H08 319206 A (SUMITOMO CHEM CO LTD) 3 December 1996 (1996-12-03)

## Description

### Field of the invention

This invention lies within the general field of chemistry and refers in particular to a synergistic surfactant composition.

### State of the art

wetting agents" are substances that lower the surface tension of water and "superwetting agents" are those substances that reduce surface tension of water below a value of 25 mN/m.

Currently, surfactants are widely used in most industries (cosmetic, agriculture, pharmaceuticals, et.) as described in patent US6653288 and in patent application CN102440250.

However, despite the large number of compounds that act as surfactants, reducing surface tension below 25 mN/m is very complicated with substances that are not organosilicones.

There is therefore a need to provide a composition that has a "super-wetting" effect and that can lower surface tension to below 25 mN/m.

The document US2004/221765 discloses an improved polytetrafiluoroethylene dispersion in water. Specifically, the inventors have discovered that polyoxyalkoxylated silicon glycol surfactants, when added to aqueous polytetrafluoroethylene dispersions, produce improved dispersion characteristic. Preferred polyoxyalkoxylated silicon glycol surfactants have low viscosities of about 30 to 60 centistokes when measured at 25°. Glycerol also produces improved aqueous dispersions of polytetrofluoroethylene and works synergistically with the foregoing polyoxyalkoxylated silicone glycol surfactants to produce optimum results. The foregoing improved dispersions are particularly suited for use in electroless plating baths such as electroless nickel plating baths. Use of the foregoing improved dispersions in electroless plating baths yield plated deposits with increased dispersion content.

### Description of the invention

The invention solves the problem of the prior art with the features disclosed in the claim 1. More concretely, the problem is solved with a synergistic composition of surfactants consisting of 10%-90% by weight of glycerine; 10%-70% by weight of monopropyleneglycol and 1%-30% by weight of organosilicone. In an embodiment of the composition, the glycerine is obtained from the synthesis of biodiesel.

In one aspect of the invention, it is disclosed a method for obtaining the previously disclosed composition comprising the steps of: (a) obtaining unrefined glycerine from the synthesis of biodiesel; (b) mixing the glycerine with organosilicon and monopropyleneglycol by simple addition and homogenization of a composition of surfactants consisting of 10%-90% by weight of glycerine; 10%-70% by weight of monopropyleneglycol and 1 %-30% by weight of organosilicone; and (c) wherein the addition is independent of the order in which the glycerine, monopropyleneglycol and organosilicone are added.

Finally, the afore-mentioned composition can be used as a super-wetting agent, wherein a super-wetting agent is understood to refer to those substances that lower the surface tension of water below a value of 25 mN/m. The composition, in another embodiment, can be used as an an insecticide, herbicide, fungicide, food, bio stimulant or fertilizer.

### Description of the figures

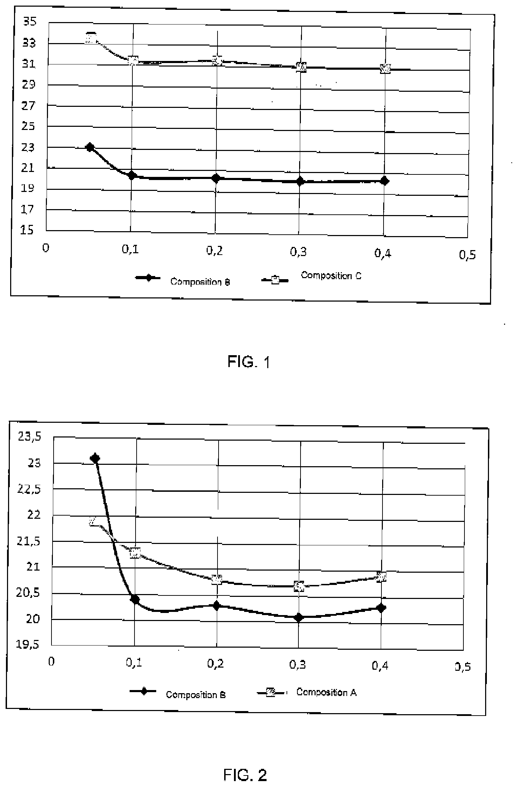

Figure 1 shows the surface tension reduction graph of composition B and of composition C. The vertical axis shows the surface tension value of the water solution in mN/m and the horizontal axis shows the concentration value as % p/p of composition B or composition C dissolved in water.
Figure 2 shows the surface tension reduction graph of composition B and of composition A. The vertical axis shows the surface tension value of the water solution in mN/m and the horizontal axis shows the concentration value as % p/p of composition B or composition A dissolved in water.

### Detailed description of the invention

### Example 1: Synergistic effect of the compositions of this invention.

To prepare the composition of this invention, unrefined glycerine from the synthesis of bio Diesel was used and was mixed with organosilicone and monopropyleneglycol (MPG). The mixing was produced by simple addition and homogenising of the three components, the preparation of the product being independent of the order in which they are added.

To check the synergistic effect, the surface tension was measured of each individual component, combined two by two and finally the three components of the product together.

The surface tension was measured by preparing the appropriate solutions with mains water and measuring the surface tension value at room temperature (25±2 °C) in a Kruss K9ET plate tensiometer (Wilhelmy platinum plate method). This method is based on measuring the maximum force needed in the vertical direction on a plate in contact with the surface of a liquid in the measuring container so that the liquid separates from the surface. This plate is connected to electronic scales to determine the surface tension value.

The measurement process involves the following steps:
- Fill the sample container with the sample to be analysed and place it in the tensiometer equipped with a platinum plate connected to the scales.
- Raise the sample container until the surface of the liquid is just below the bottom face of the plate.
- Rate the equipment.
- Continue to raise the sample container slowly until the plate makes first contact with the surface of the liquid. Stop it as soon as there is a first contact with the liquid.
- Turn the gauge wheel until the wheels indication matches the fixed mark on the equipment. This leaves a reference of the point at which the first contact occurred between the sample and the platinum plate.
- Raise the sample 5 mm, submerging the plate and checking that the plate is wetted with the product to be analysed.
- Lower the sample container slowly to remove the plate from the product solution until the reference mark aligns with the fixed mark on the equipment again (first contact of the platinum plate with the sample).

The results are shown in the following tables.

**Table 1: Reduction of surface tension values at concentrations of 0.025% p/p.**

| Glycerine | MPG | Organosilicone | Surface tension (mN/m) |
|---|---|---|---|
| 0.025 %p/p | | | 71.7 |
| | 0.025 %p/p | | 72,3 |
| | | 0.025 %p/p | 29.0 |
| 0.025 %p/p | | 0.025% p/p | 28.6 |
| | 0.025 %p/p | 0.025% p/p | 28.9 |
| 0.025 %p/p | 0.025 %p/p | | 68.1 |
| 0,025 %p/p | 0.025 %p/p | 0.025 %p/p | 28.7 |

**Table 2: Reduction of surface tension values at concentrations of 0.050% p/p.**

| Glycerine | MPG | Organosilicone | Surface tension (mN/M) |
|---|---|---|---|
| 0.050 %p/p | | | 68.7 |
| | 0.050 %p/p | | 70.3 |
| | | 0.050 %p/p | 25.2 |
| 0.050 %p/p | | 0.050 %p/p | 24.1 |
| | 0.050 %p/p | 0.050 %p/p | 24.3 |
| 0.050 %p/p | 0.050 %p/p | | 66.0 |
| 0.050 %p/p | 0.050 %p/p | 0.050 %p/p | 22.5 |

**Table 3: Reduction of surface tension values at concentrations of 0,100% p/p.**

| Glycerine | MPG | Organosilicone | Surface tension (mN/m) |
|---|---|---|---|
| 0.100 %p/p | | | 67.0 |
| | 0.100 %p/p | | 69.5 |
| | | 0.100 %p/p | 20.9 |
| 0**.**100 %p/p | | 0.100 %p/p | 22.2 |
| | 0,100 %p/p | 0.100 %p/p | 22.9 |
| 0.100 %p/p | 0.100 %p/p | | 70.6 |
| 0.100 %p/p | 0.100 %p/p | 0.100 %p/p | 20.3 |

As can be seen in Tables 1-3, the effect of the organosilicone prevails over the other individual components at equal concentrations. Even so, an additional reduction of surface tension is achieved to reach 20.3 mN/m, taking advantage of the synergistic effect of the mixture of the three components. Note that it is necessary to use a combination of three components to achieve an additional reduction of surface tension of approximately 10%, compared to organosilicone. alone.

To confirm the effect of the reduction of surface tension of composition B and that this effect is maintained in the presence of the three components, two different compositions for the product were tested and the effect of these new formulas (B1 and B2) measured.

**Table 4: Table for composition of formulas B and variations B1 and B2.**

| **Composition** | **Glycerine (%p/p)** | **Monopropyleneglycol (%p/p)** | **Organosilicone (%p/p)** |
|---|---|---|---|
| B | 56 | 34 | 10 |
| B1 | 53 | 32 | 15 |
| B2 | 58 | 35 | 7 |

Surface tension at 0.2% p/p:
B: 20.3 mN/m
B1: 20.2 mN/m
B2:20.6 mN/m

At this point it can be seen that composition B is the optimal one, taking into account the compromise between the synergistic effect seen (towering of surface tension) and the amount of organosilicone to be used.

The composition of this invention (composition B) was then compared with other compositions (composition A and composition C) containing organosilicone mixed with other non-ionic surfactants. In fact, it is known that these substances can improve the effectiveness of organosilicone but from the point of view of reducing the dose.

Table 5 shows that the composition of this invention not only improves the effectiveness in the sense of reducing the dose but also improves it in the sense of achieving a reduction in the surface tension of water, in absolute value terms, attaining a lower surface tension value than that achieved with organosilicone alone or using other potentially synergistic substances in the general sense.

It is thus shown that the new superwetting and synergistic effect is due to the mix of the three compounds of this invention.

**Table 5: Reduction of surface tension values compared with other products containing organosilicone.**

| | Solution at 0.1% in water | Solution at 0.2% in water | Solution at 0.3% in water |
|---|---|---|---|
| Composition | Surface tension (mN/m) | Surface tension (mN/m) | Surface tension (mN/m) |
| A (repetition 1) | 21.4 | 20.8 | 20.8 |
| A (repetition 2) | 21.3 | 20.9 | 20.7 |
| B (repetition 1) | 20.4 | 20.3 | 20.0 |
| B (repetition 2) | 20.5 | 20.4 | 20.2 |
| C (repetition 1) | 31.4 | 31.7 | 31.0 |
| C (repetition 2) | 31.6 | 31.5 | 31.1 |

Composition A, containing 60% organosilicone, 30% monoethylenglycol and 10% sodium lauryl sulphate.

The composition of this invention, containing 56% glycerine, 34% monopropylaneglycol and 10% organosilicone.

Composition C consisted of 90% canola oil and 10% organosilicone.

As shown by the results in Tables 4 and 5, the composition of this invention (composition B) shows the best reduction of surface tension. Composition B generates lower surface tension values than combination A, although it contains a sixth less of the same organosilicone.

When diluted in water with the same dose (0.2%), composition B generates the same range of water surface tension reduction as composition A. It is important to note that at surface tension levels approaching 20 mN/m, achieving a reduction of 0.5 mN/m is really complicated even though the difference in absolute value may appear small, but it must be remembered that it is not since this is very close to the cancellation of the dosage effect, The same occurs when composition B is compared with composition C.

Extending the range of concentrations comparing composition B and composition A and the same composition B compared to composition C, it can be seen that:
- compared with a product containing the same quantity of organosilicone. (combination C, 10% organosilicone), the reduction of the surface tension is significantly sharper for the case of this invention (combination B).

**Table 6: Reduction of surface tension B vs C**

| Concentration in water (%p/p) | Composition B | Composition C |
|---|---|---|
| 0.05 | 23.1 | 33.6 |
| 0.10 | 20.4 | 31.5 |
| 0.20 | 20.3 | 31.6 |
| 0.30 | 20.1 | 31.1 |
| 0.40 | 20.3 | 31.1 |

- compared with composition A (with a concentration six times greater of organosilicone) it can be seen that a concentration of 0.1 % p/p in water has already exceeded the effect of composition A and maintains this reduction at higher concentrations.

**Table 7: Reduction of surface tension B vs A**

| Concentration in water (%p/p) | Invention (B) | Other A |
|---|---|---|
| 0.05 | 23.1 | 21.9 |
| 0.10 | 20.4 | 21.3 |
| 0.20 | 20.3 | 20.8 |
| 0.30 | 20.1 | 20.7 |
| 0.40 | 20.3 | 20,9 |

It should be noted that with composition A, water surface tension values below 20.5 mN/m are never generated. On the other hand, all the values generated by composition B are bellow this value.

## Claims

1. A synergistic composition of surfactants consisting of 10%-90% by weight of glycerine; 10%-70% by weight of monopropyleneglycol and 1%-30% by weight of organosilicone.

2. The composition of claim 1 wherein the glycerine is obtained from the synthesis of biodiesel.

3. A method for obtaining the composition of claims 1 and 2 comprising the steps of:
a. obtaining unrefined glycerine from the synthesis of biodiesel; and
b. mixing the glycerine with organosilicon and monopropyleneglycol by simple addition and homogenisation of a composition of surfactants consisting of 10%-90% by weight of glycerine; 10%-70% by weight of monopropyleneglycol and 1%-30% by weight of organosilicone; and
c. wherein the addition is independent of the order in which the glycerine, monopropyleneglycol and organosilicone are added.

4. The composition of claims 1 or 2 for use as a super-wetting agent.

5. The composition of claims 1 or 2 for use as an insecticide, herbicide, fungicide, food, bio stimulant or fertiliser.

## Patentansprüche

1. Synergistische Zusammensetzung von Tensiden, bestehend aus 10-90 Gewichtsprozent Glyzerin; 10-70 Gewichtsprozent Monopropylenglykol und 1-30 Gewichtsprozent Organosilikon.

2. Zusammensetzung nach Anspruch 1, wobei das Glyzerin aus der Synthese von Biodiesel gewonnen wird.

3. Methode zur Erzeugung der Zusammensetzungen aus den Ansprüchen 1 und 2, die folgende Schritte umfasst:
a. Gewinnen von unraffiniertem Glyzerin aus der Synthese von Biodiesel; und
b. Mischen des Glyzerins mit Organosilikon und Monopropylenglykol durch einfache Zugabe und Homogenisierung einer Zusammensetzung von Tensiden, bestehend aus 10-90 Gewichtsprozent Glyzerin; 10-70 Gewichtsprozent Monopropylenglykol und 1-30 Gewichtsprozent Organosilikon; und
c. wobei die Zugabe unabhängig von der Reihenfolge ist, in der Glyzerin, Monopropylenglykol und Organosilikon zugegeben werden.

4. Zusammensetzung nach den Ansprüchen 1 oder 2 zur Verwendung als starkes Benetzungsmittel.

5. Zusammensetzung nach den Ansprüchen 1 oder 2 zur Verwendung als Insektizid, Herbizid, Fungizid, Nahrungsmittel, Biostimulans oder Düngemittel.

## Revendications

1. Une composition synergique de tensioactifs consistant en 10 %-90 % en poids de glycérine ; 10 %-70 % en poids de monopropylèneglycol et 1 %-30 % en poids d'organosilicone.

2. La composition selon la revendication 1 où la glycérine est obtenue à partir de la synthèse de biodiesel.

3. Une méthode pour obtenir la composition selon les revendications 1 et 2 comprenant les étapes de :
a. obtention de glycérine non raffinée à partir de la synthèse de biodiesel ; et
b. Mélange de glycéwrine avec de l'organosilicone et du monopropylèneglycol par simple ajout et homogénéisation d'une composition de tensioactifs consistant en 10 %-90 % en poids de glycérine ; 10 %-70 % en poids de monopropylèneglycol et 1 %-30 % en poids d'organosilicone ; et
c. où l'ajout est indépendant de l'ordre dans lequel la glycérine, le monopropylèneglycol et l'organosilicone sont ajoutés.

4. La composition des revendications 1 ou 2 pour l'utilisation en tant qu'agent super-mouillant.

5. La composition des revendications 1 ou 2 pour l'utilisation en tant qu'insecticide, herbicide, fongicide, aliment, biostimulant ou fertilisant.
